# EUROPEAN PATENT APPLICATION

(11) **EP 2 228 042 A1**
(43) Date of publication of application: **15.09.2010**
(21) Application number: 08866000.6
(22) Date of filing: 25.12.2008
(51) Int. Cl.: A61F 2/44, A61F 2/30, A61L 27/00

(54) **BIOMATERIAL FOR ARTIFICIAL CARTILAGES**

(30) Priority: 28.12.2007 JP 2007339361
(71) Applicant: TAKIRON CO., LTD., Osaka-shi Osaka 541-0052 (JP)
(72) Inventor: SHIKINAMI, Yasuo, Osaka-shi Osaka 541-0052 (JP)
(74) Representative: HOFFMANN EITLE
(86) International application number: PCT/JP2008/073516
(87) International publication number: WO 2009/084559

(57) **Abstract**

An object of the present invention is to provide a biomaterial for an artificial cartilage that eliminates the risk of possible adverse effects due to scattering of fragments of the biodegradable and bioabsorbable polymers and is joined further solidly to vertebral bodies and the like with increased adhesiveness.

The biomaterial for an artificial cartilage according to the present invention includes: an organized structure comprising organic fibers arranged in one of a multiaxial three-dimensional woven or knitted structure having three or more axes and a combined structure of the woven structure and the knitted structure; and a biodegradable and bioabsorbable pin provided inside the organized structure, two ends of the pin becoming projected from upper and lower surfaces of the organized structure upon compression on the organized structure from above and below, each of the upper and lower surfaces or either one of the surfaces of the organized structure having a superficial portion of a soft layer that is softer than the other portion of the organized structure, part or all of the organic fibers of the superficial portion being coated with a biodegradable and bioabsorbable polymer complex containing bioactive bioceramic powder. Adhesiveness with vertebral bodies and the like is increased by making the superficial portion(s) of the organized structure as a soft layer, the scattering of the polymers is prevented by coating the organic fibers of the superficial portion with the above complex, and the solid joint to vertebral bodies and the like is achieved.

## Description

### TECHNICAL FIELD

The present invention relates to biomaterials for artificial cartilages expected for use as artificial intervertebral discs, artificial menisci, and various fibrous articular cartilages.

### BACKGROUND ART

Regarding the above-mentioned type of biomaterials for artificial cartilages, the present applicant has proposed a biomaterial including a core material constructed from an organized structure that comprises organic fibers arranged in one of a multiaxial three-dimensional woven or knitted structure having three or more axes and a combined structure of the woven structure and the knitted structure, a porous plate of a biodegradable and bioabsorbable polymer containing bioactive bioceramic powder, the plate being laminated on either one or each of the surfaces of the core material, and pins that are passed through the core material and the plate(s) such that two ends of each pin project therefrom (Patent Document 1).

The biomaterial for an artificial cartilage is a stand-alone biomaterial capable of being fixed in an interbody without causing misalignment or dissociation by means of the pins, both ends of the pins slightly sticking into the superior and inferior vertebral bodies when the biomaterial is inserted as, e.g., an artificial intervertebral disc between the superior and inferior vertebral bodies. Further, with the progress of hydrolysis of the plates that are laminated on the core material, biogenic bone tissue conductively (inductively) grows into the plates because of the osteoconductivity or osteoinductivity of the bioceramic powder, so that the plates are finally replaced with the bone tissue to provide direct joint and fixation to the vertebral bodies. Since the core material constructed from the organized structure of organic fibers has mechanical strength and flexibility (behavior) that simulate cartilages such as intervertebral discs and the deformation thereof is highly biomimetic, the biomaterial is very useful, providing a satisfactory function as an intervertebral disc.
Patent Document 1: JP-A-2005-143800

### DISCLOSURE OF THE INVENTION

### PROBLEMS TO BE SOLVED BY THE INVENTION

The biomaterial for an artificial cartilage of Patent Document 1 causes no particular problem as long as the biomaterial to be used has a size matched for the interbody into which the biomaterial is to be inserted and installed. If, however, the biomaterial for an artificial cartilage has a size (especially the height and shape) which is not matched to the interbody space, a gap is formed between the plates and the vertebral bodies, which causes stress on the plates in a complicated manner owing to the pressure and movement of the vertebral bodies and possibly leads to breakage of the plates. The breakage of the plates and scattering of fragments thereof may invite a possible adverse effect, such as stimulation on nerve roots or dural medullary space; hence, an effective measure against the problem is called for.

The present invention was made in view of the foregoing circumstances, and it is an object of the invention to provide a biomaterial for an artificial cartilage that, while preserving the benefits of the biomaterial for an artificial cartilage of Patent Document I, eliminates the risk of possible adverse effects due to scattering of fragments of the plates and is joined further solidly to biogenic bones such as vertebral bodies with increased adhesiveness, thereby obviating the problem of migration or dissociation from the installed position.

### MEANS FOR SOLVING THE PROBLEMS

In order to overcome the problem, a biomaterial for an artificial cartilage according to the present invention includes: an organized structure comprising organic fibers arranged in one of a multiaxial three-dimensional woven or knitted structure having three or more axes and a combined structure of the woven structure and the knitted structure; and a biodegradable and bioabsorbable pin provided inside the organized structure, two ends of the pin becoming projected from upper and lower surfaces of the organized structure upon compression on the organized structure from above and below, each of the upper and lower surfaces or either one of the surfaces of the organized structure having a superficial portion of a soft layer that is softer than the other portion of the organized structure, part or all of the organic fibers of the superficial portion being coated with a biodegradable and bioabsorbable polymer complex containing bioactive bioceramic powder.

In the biomaterial for an artificial cartilage according to the present invention, it is preferred that the layer coated with the biodegradable and bioabsorbable polymer complex has a thickness in a range of 0.2 to 3 mm, that the superficial portion on the upper surface of the organized structure is approximately as soft as or softer than the superficial portion on the lower surface, that at least the superficial portion on the upper surface comprises a plurality of soft layers and the uppermost soft layer thereof is the softest, and that each of the superficial portions on the upper and lower surfaces or the superficial portion on either one of the surfaces of the organized structure is raised or recessed in such a manner as to follow the terrain of a contact surface of a living body.

In the biomaterial for an artificial cartilage according to the present invention, it is preferred that the biodegradable and bioabsorbable polymer complex contains the bioceramic powder by 50 to 90 percent by volume, and that the biodegradable and bioabsorbable polymer is any ofpoly(D,L-lactic acid), a copolymer of L-lactic acid and D,L-lactic acid, a copolymer of lactic acid and glycolic acid, a copolymer of lactic acid and p-dioxanone, a copolymer of lactic acid and ethylene glycol, and a copolymer of lactic acid and caprolactone that is used alone or as a mixture of two or more thereof; however, a restrictive condition is set with respect to the composition of the biodegradable and bioabsorbable polymer complex such that the bioceramic powder (the particle diameter is equal to or less than 30 µm, preferably equal to or less than 10 µm, more preferably on the order of 0.1 to 5 µm) shall not separate easily from the complex even when the ratio of the powder is high, and that such a situation shall be avoided that, when the polymer complex is coated over the fibers, the polymer at an excessive ratio causes hardening of the polymer complex and thus impairs the flexibility. Although varied depending on the particle diameter, the condition is met in the case where the bioceramic powder is contained in the biodegradable and bioabsorbable polymer in a range of 50 to 90 percent by volume.

While the biomaterial for an artificial cartilage according to the present invention is applicable as, e.g., an artificial intervertebral disc, an artificial meniscus, and various articular cartilages, the biomaterial is typically used as an artificial intervertebral disc that is inserted and installed in an interbody.

### EFFECTS OF THE INVENTION

When the biomaterial for an artificial cartilage of the present invention is inserted between superior and inferior vertebral bodies as, e.g., an artificial intervertebral disc, both the ends of the biodegradable and bioabsorbable pin become projected from the upper and lower surfaces of the organized structure that is receiving compression from above and below by the superior and inferior vertebral bodies and the ends stick slightly into the superior and inferior vertebral bodies, so that the biomaterial is fixed between the superior and inferior vertebral bodies in a free-standing manner without causing misalignment or dissociation. Then, the soft layer (soft superficial portion) on each or either one of the upper and lower surfaces of the organized structure compressed changes its shape easily to bulge and sink along the terrain of the contact surface of each of or either one of the superior and inferior vertebral bodies into adhesion with the vertebral body. In the biomaterial for an artificial cartilage thus fixed in a free-standing manner between the superior and inferior vertebral bodies into adhesion with the vertebral bodies, the organized structure that comprises organic fibers arranged in one of a multiaxial three-dimensional woven or knitted structure having three or more axes and a combined structure of the woven structure and the knitted structure exhibits mechanical strength, flexibility, and dynamic deforming behavior that are similar to those possessed by cartilages such as intervertebral discs, and therefore the biomaterial follows the movement of the vertebral bodies and deforms highly biomimetically, thereby providing a satisfactory function as an artificial intervertebral disc. It has been confirmed that this behavior that mimics the movement of a living body is maintained for fifty years.

Meanwhile, the bioceramic powder-containing biodegradable and bioabsorbable polymer complex that is coated over the organic fibers of the superficial portion of each or either one of the upper and lower surfaces of the organized structure comes in contact with humor that has permeated through the superficial portion and the hydrolysis of the polymer complex progresses; the bioceramic powder that is gradually released and exposed over the hydrolysis exerts osteoinductivity or osteoconductivity to induce (conduct) bone tissue to grow into the superficial portion of the organized structure from a vertebral body. At a final stage, the biodegradable and bioabsorbable polymer is replaced with the bone tissue and the superficial portion of the organized structure is solidly joined and fixed to the vertebral body. After the organized structure and the vertebral bodies are solidly joined to each other, the pin also degrades and is absorbed until disappearance. On the other hand, the organized structure has an inner layer portion that is not coated with the biodegradable and bioabsorbable polymer complex, and since the organic fibers, such as polyethylene fiber, are non-bioactive in itself, the inner layer portion is not subject to invasion of bone tissue (although partial invasion of fibrous tissue is possible) and thus will not be hardened, because of which the original dynamic deforming behavior is maintained almost permanently.

The biomaterial for an artificial cartilage of the present invention is not structured such that plates of a bioceramic powder-containing biodegradable and bioabsorbable polymer complex are laminated on the two surfaces; instead, all or part of the organic fibers of the soft superficial portion(s) of the organized structure are coated with a bioceramic powder-containing biodegradable and bioabsorbable polymer complex. Moreover, the organized structure exhibits biomimetic dynamic deforming behavior and the soft layer (soft outer surface layer) on each or either one of the upper and lower surfaces of the organized structure tightly mates with the vertebral body. Thus, while undergoing degradation, the biodegradable and bioabsorbable polymer complex does not generate fragments (debris) by the pressure or movement of the vertebral bodies, and therefore it is possible to eliminate the risk of possible harmful effects such as affecting (inducing rejection against foreign substances) on nerve roots or dural medullary space due to scattering of the fragments (debris). When the soft layer(s) (soft outer surface layer(s)) of the organized structure thus coated with the biodegradable and bioabsorbable polymer complex adhere(s) to the vertebral body (bodies), bone tissue rapidly grows inductively (conductively) into the superficial portion(s) of the organized structure, such that the bone tissue that has ingrown inductively (conductively) become intertwined with the organic fibers of the superficial portion(s), thereby allowing the organized structure and the vertebral bodies to be joined early with great joining strength.

A biomaterial for an artificial cartilage of the present invention that has a 0.2 to 3 mm-thick layer coated with the biodegradable and bioabsorbable polymer complex in a superficial portion of the organized structure is capable of joining solidly to vertebral bodies after being inserted and installed in an interbody as described above. Moreover, since biogenic bone tissue does not grow inductively (conductively) into the inner layer portion (core layer portion) of the organized structure and the inner layer portion of the organized structure does not become hardened, the biomaterial maintains the biomimetic dynamic deforming behavior and is thus capable of functioning satisfactorily as an artificial intervertebral disc. If the thickness of the coating layer in the superficial portion is less than 0.2 mm, the bone tissue to be formed inductively (conductively) will have small layer thickness and the organic fibers and the bone tissue will not be intertwined sufficiently, hence providing reduced joining strength between the organized structure and the vertebral bodies. On the other hand, the coating layer in the superficial portion having a thickness greater than 3 mm leads to relative reduction in thickness of the inner layer portion that will not have bone tissue ingrown inductively (conductively) therein, which will impede the physical behavior required for an artificial intervertebral disc and hence hinder the biomimetic deformation of the organized structure.

Consider the situation in which the biomaterial for an artificial cartilage of the present invention is inserted and installed between superior and inferior human vertebral bodies. The inferior surface of the vertebral body to be pressingly contacted by the upper surface of the organized structure (the inferior surface of the superior vertebral body) is normally recessed deeply as compared with the superior surface of the vertebral body to be pressingly contacted by the lower surface of the organized structure (the superior surface of the inferior vertebral body), and the superior surface of a vertebral body is normally almost flat as compared with the inferior surface of the vertebral body. Accordingly, in the case of a biomaterial for an artificial cartilage of the present invention having, on the upper surface of the organized structure, a superficial portion that is approximately as soft as or softer than the superficial portion on the lower surface, the superficial portion on the upper surface of the organized structure is capable of deforming greatly to conform to the deeply recessed geometry of the inferior surface of the vertebral body, thereby providing increased adhesion to the inferior surface of the superior vertebral body. While being less in flexibility (softness) than the superficial portion on the upper surface, the superficial portion on the lower surface of the organized structure is capable of deforming a little to conform to the approximately flat geometry of the superior surface of the vertebral body, thereby providing adhesion to the superior surface of the inferior vertebral body.

In the case of a biomaterial for an artificial cartilage in which at least the superficial portion on the upper surface of the organized structure comprises a plurality of soft layers and the uppermost soft layer is the softest, upon insertion and installation of the biomaterial between superior and inferior vertebral bodies, the plurality of soft layers constituting the superficial portion on the upper surface, as a whole, follow the deeply recessed inferior surface of the superior vertebral body and deform their shapes greatly while the softest uppermost layer deforms a little to conform to the fine concave/convex terrain on the deeply recessed inferior surface of the vertebral body, thereby providing further increased adhesion to the inferior surface of the vertebral body.

Moreover, in the case of a biomaterial for an artificial cartilage in which the superficial portion on each of or either one of the upper and lower surfaces of the organized structure is raised or recessed so as to follow the terrain of a contact surface of a living body, such as the superior or inferior surface of a vertebral body, when the biomaterial is inserted and installed between superior and inferior vertebral bodies with, e.g., the raised superficial portion facing upward, the raised superficial portion substantially matches and flexibly meets the recessed inferior surface of the superior vertebral body, which surface has a shape varied from person to person, whereby the pressure from the superior vertebral body is applied substantially uniformly onto the organized structure, and besides the raised superficial portion functions to prevent misalignment or dissociation of the organized structure. Meanwhile, since the superior surface of the vertebral body to contact the lower surface of the organized structure normally has an almost flat shape in comparison with the inferior surface as described above, the outer surface layer on the lower surface of the organized structure need not be so raised (or recessed, depending on the circumstances) as the outer surface layer on the upper surface is, and it may be flat.

In the case of a biomaterial for an artificial cartilage of the present invention in which the biodegradable and bioabsorbable polymer complex to be coated contains the bioceramic powder by 50 to 90 percent by volume, bone tissue is rapidly formed inductively (conductively) into a superficial portion of the organized structure by the action of the osteoinductivity or osteoconductivity of the appropriate amount of bioceramic powder, so that the organized structure and the vertebral bodies are joined to each other early. If the content of the bioceramic powder is less than 50 percent by volume, the inductive (conductive) growth rate of bone tissue is slow because of the insufficient amount of the bioceramic powder, whereas if the content is more than 90 percent by volume, the excessive amount of bioceramic powder may spill from the superficial portion of the organized structure; therefore, neither content is preferred.

In the case of a biomaterial for an artificial cartilage of the present invention in which, e.g., the aforementioned poly(D,L-lactic acid) is used as a biodegradable and bioabsorbable polymer of the biodegradable and bioabsorbable polymer complex to be coated, the polymer is resilient and is not brittle, in addition to being relatively fast in hydrolysis, having amorphous or crystalline-amorphous mixed constitution; hence, fragments are not likely to be produced due to the pressure and movement of vertebral bodies, hydrolysis proceeds fast, causing the bioceramic powder to be exposed, and bone tissue is rapidly formed inductively (conductively) into the superficial portion of the organized structure to attain joint with the vertebral bodies. Accordingly, such a molecular weight is required that the bioceramic powder is retained with a certain degree of strength, and so the molecular weight is 10,000 to 100,000, preferably 30,000 to 50,000 (viscosity-average molecular weight) in the case of, e.g., poly(D,L-lactic acid).

### BRIEF DESCRIPTION OF THE DRAWINGS

Fig. 1 is a perspective view of a biomaterial for an artificial cartilage according to one embodiment of the present invention.
Fig. 2 is an illustrative view showing the biomaterial in use.
Fig. 3 is a schematic illustrative view of the biomaterial.
Fig. 4A is a schematic illustrative view showing a state in which the biomaterial is inserted into an opened interbody, Fig. 4B is a schematic illustrative view showing a state in which the biomaterial is compressed with the pressure from superior and inferior vertebral bodies, and Fig. 4C is a schematic illustrative view showing a state in which the biomaterial is deformed by the anteroposterior bending of the vertebral bodies.
Fig. 5 is a perspective view of a biomaterial for an artificial cartilage according to another embodiment of the present invention.
Fig. 6 is a perspective view of a biomaterial for an artificial cartilage according to still another embodiment of the present invention.
Fig. 7 is a plan view showing positions in which the biomaterials for an artificial cartilage are inserted.
Fig. 8 is a schematic illustrative view of a biomaterial for an artificial cartilage according to still another embodiment of the present invention.

### DESCRIPTION OF REFERENCE NUMERALS

- 1: organized structure of organic fibers
- 1a: superficial portion (soft layer)
- 2: biodegradable and bioabsorbable pin
- 3: biodegradable and bioabsorbable polymer complex containing bioactive bioceramic powder
- 10, 11, 12, 13: biomaterial for artificial cartilage
- 11a a: soft layer (upper superficial portion)
- 12a: soft layer (lower superficial portion)
- 20: vertebral body

### BEST MODE FOR CARRYING OUT THE INVENTION

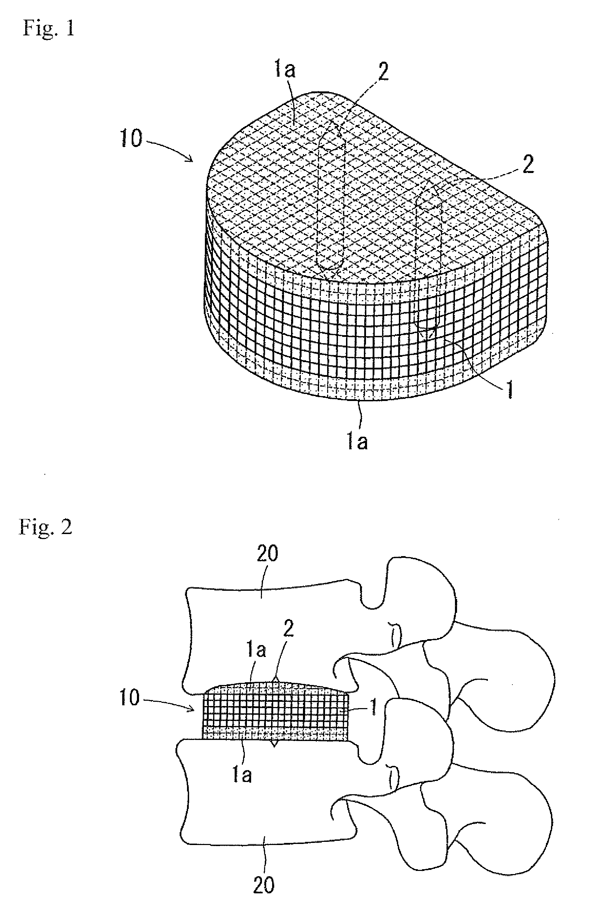

A biomaterial for an artificial cartilage according to one embodiment of the present invention is described with reference to Figs. 1 to 4C.

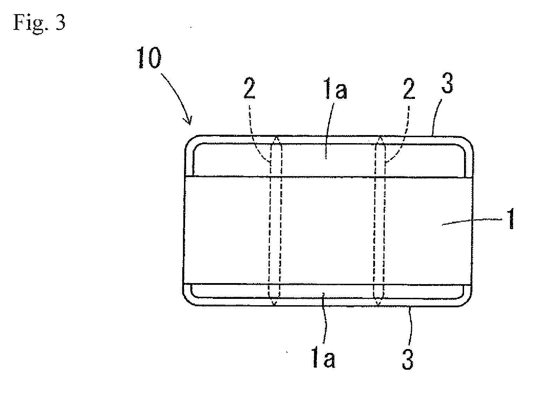

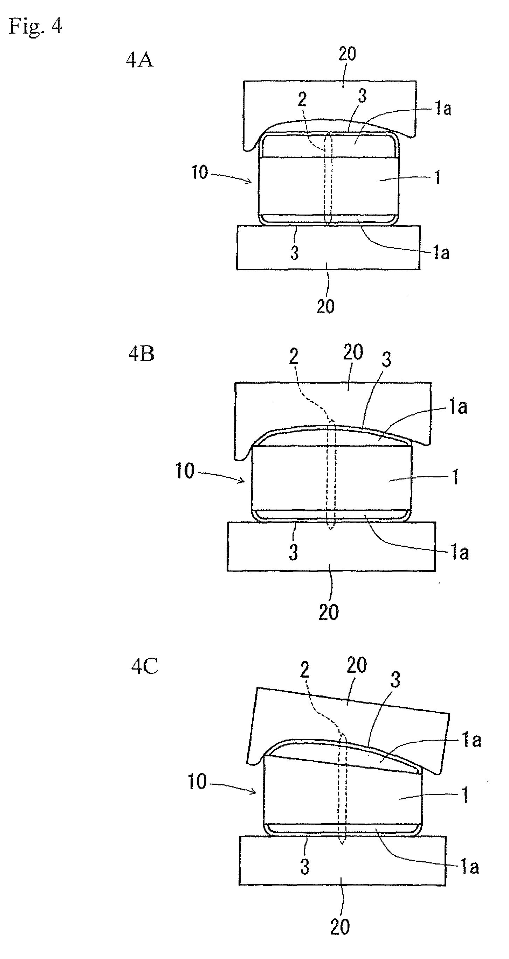

As shown in Figs. 2 and 4A to 4C, the biomaterial 10 for an artificial cartilage is inserted and installed as a whole replacement type artificial intervertebral disc between superior and inferior vertebral bodies 20. As shown in Fig. 1, the biomaterial is constructed from an organized structure 1 of organic fibers with its anterior half having a semicircular shape and posterior half having a rectangular shape. A plurality of (two in the present embodiment) pins 2 are provided in a vertical direction inside the organized structure 1. Both the ends of the pins 2 are buried in the organized structure 1 and do not project from the upper and lower surfaces of the organized structure, e.g., in a state where the biomaterial is yet to be inserted into an interbody as shown in Figs. 1 and 3 or in a state where the biomaterial is put between the vertebral bodies 20 without being compressed as shown in Fig. 4A. When the organized structure 1 receives compression from the superior and inferior vertebral bodies 20 following the insertion of the biomaterial, as shown in Figs. 2, 4B, and 4C, both the ends of the pins 2 project from the upper and lower surfaces of the organized structure 1 to stick into the superior and inferior vertebral bodies 20, and superficial portions 1a on the upper and lower sides, which portions are formed as soft layers, are deformed in conformity to the surface terrains of the vertebral bodies 20 and are reduced to smaller thicknesses, so that the biomaterial is fixed between the vertebral bodies 20 in a free-standing manner without being misaligned or dissociated. The number of the pins 2 is typically two in the case of using the biomaterial for a cervical vertebra and three in the case of using the biomaterial for a lumbar vertebra.

The size of the biomaterial 10 for an artificial cartilage for use as an artificial intervertebral disc is varied depending on whether it is used for adults or children, or for vertebral columns (lumbar vertebrae) or cervical vertebrae; in the case of a biomaterial used for the vertebral column of an adult, the lateral width dimension is on the order of 30 to 40 mm, the anteroposterior dimension is on the order of 25 to 30 mm, and the thickness is on the order of 10 to 15 mm.

The organized structure 1 constituting the biomaterial 10 for an artificial cartilage comprises organic fibers that are arranged in either one of a three-dimensional woven or knitted structure and a combined structure of the woven structure and the knitted structure. The organized structure is nearly equal in mechanical strength and flexibility to cartilages such as intervertebral discs and is capable of deforming in a highly biomimetic fashion.

The organized structure 1 is approximately the same as the organized structure described in Japanese Patent Application No. 06-254515 (Japanese Patent No. 3243679) that was filed by the present applicant, and preferably used is a structural body with a multiaxial and three-dimensional structure having three or more axes, where the geometrical shape is represented by the number of dimensions and the number of orientations of fiber arrangement is represented by the number of axes.

The three-axial and three-dimensional structure is constructed such that fibers extending in three axial directions, i.e., longitudinal, lateral, and vertical directions, are arranged three-dimensionally, and the structural body typically has a bulk (planar or block-like) shape with a certain thickness as mentioned above; however, a cylindrical shape and a honeycomb shape may also be employed. Three-axial and three-dimensional structures of this type are classified into, e.g., an orthogonal structure, a non-orthogonal structure, a leno structure, and a cylindrical structure, according to the structural differences. A four-or-more-axial and three-dimensional structure enhances the isotropy of the structural body in terms of strength by means of arranging fibers in orientations along 4, 5, 6, 7, 9, 11 axes and the like. Through appropriate selection, it is possible to obtain an even more biomimetic organized structure 1 that further closely simulates a natural cartilage tissue. By devising a technique for forming such a woven or knitted structure, it becomes possible to construct an organized structure 1 with a multilayer structure wherein an organized structure 1 which has various static, dynamic, and physical properties desired for an intervertebral disc includes, on its upper and lower surfaces, superficial portions 1a that are softer than the organized structure and have surface terrains to directly contact convex, concave, or flat surfaces of vertebral bodies. An effective method to form such superficial portions 1a is, e.g., thinning down threads along a Z axis of the threads along X, Y, and Z axes or reducing the number of the threads.

The internal void ratio of the organized structure 1 is preferably in a range of 20 to 90%. If the ratio is below 20%, the organized structure 1 is fine and dense and less in flexibility and deformability and thus is not used satisfactorily as a biomaterial for an artificial cartilage. If the ratio is above 90%, the organized structure 1 degrades in compressive strength and ability to retain the shape, thus also resulting in a material inappropriate for use as a biomaterial for an artificial cartilage.

The organic fibers constituting the organized structure 1 are preferably a bioinert synthetic resin fiber of, e.g., polyethylene, polypropylene, or polytetrafluoroethylene, or a bioinert coated fiber that is obtained by coating an organic core-fiber with the aforementioned bioinert resins. In particular, a coated fiber that is on the order of 0.2 to 0.5 mm in diameter and is obtained by coating a core-fiber of a ultrahigh-znolecular polyethylene with a linear low-density polyethylene film is optimal in terms of strength, hardness, resilience, facility of weaving or knitting, and the like. Besides, a bioactive (e.g., osteoconductive or osteoinductive) fiber may also be chosen.

Since the organized structure 1 is disclosed in detail in Japanese Patent Application No. 06-254515, further description thereof is not given.

As shown in Fig. 3, coating layers of the biodegradable and bioabsorbable polymer complex 3 containing bioactive bioceramic powder are formed on the superficial portions 1a on the upper and lower surfaces of the organized structure 1, and part of the organic fibers (the organic fibers present along the surfaces) of the superficial portions 1a are coated with the polymer complex 3. A method of coating the organic fibers of the superficial portions 1a is, e.g., such that a biodegradable and bioabsorbable polymer is dissolved in a volatile solvent such as ethanol, dichloroethan (dichloromethan), or chloroform and bioceramic powder is mixed homogeneously therein to prepare a suspension, and then the suspension is applied to the two surfaces of the organized structure 1, or the suspension is sprayed over the two surfaces of the organized structure 1, or the two surfaces of the organized structure 1 are soaked in the suspension. Depending on the circumstances, all the organic fibers of the superficial portions 1a may be coated with the biodegradable and bioabsorbable polymer complex 3.

Suitable examples of the biodegradable and bioabsorbable polymer of the complex 3 to cover the organic fibers of the superficial portions 1a include poly(D,L-lactic acid), a copolymer of L-lactic acid and D,L-lactic acid, a copolymer of lactic acid and glycolic acid, a copolymer of lactic acid and p-dioxanone, a copolymer of lactic acid and ethylene glycol, and a copolymer of lactic acid and caprolactone that are safe, relatively fast in degradation, resilient, and not brittle, and that have an amorphous or a crystalline-amorphous mixed constitution. Any of these polymers is used alone, or two or more of these polymers are mixed for use.

The molecular weight of the polymer is not particularly limited; however, considering the strength of the coating films and the speed of degradation and absorption, one having a viscosity-average molecular weight on the order of 10,000 to 100,000 is preferably used. If the viscosity-average molecular weight is less than 10,000, the coating films are prone to peel off from the organic fibers of the superficial portions 1a due to the pressure and movement of vertebral bodies, whereas if the viscosity-average molecular weight is more than 100,000, degradation and absorption takes longer time, so that the inductive (conductive) growth of bone tissue into the superficial portions 1a proceeds at a slow rate; therefore, neither weight is preferred. The viscosity-average molecular weight of the biodegradable and bioabsorbable polymer to be coated is more preferably in a range of 30,000 to 50,000.

Preferable examples of the bioceramic powder to be contained in the biodegradable and bioabsorbable polymer complex 3 include powdery substances that are bioactive, bioabsorbable and completely replaceable with bone tissue, highly osteoinductive or osteoconductive, and highly biocompatible, such as hydroxyapatite that is uncalcined and unsintered, dicalcium phosphate, tricalcium phosphate, tetracalcium phosphate, octacalcium phosphate, calcite, ceravital, diopside, and natural coral. It is also possible to use powder obtained by attaching an alkaline inorganic compound or a basic organic substance to the surfaces of powder particles. Of these substances, hydroxyapatite that is uncalcined and unsintered, tricalcium phosphate, and octacalcium phosphate are particularly preferably used for their high bioactivity, good osteoinductivity or osteoconductivity, harmlessness, and bioabsorbability within a short period of time.

The bioceramic powder of any of these kinds to be used has a particle diameter equal to or less than 30 µm, preferably equal to or less than 10 µm, more preferably on the order of 0.1 to 5 µm*,* in view of dispersibility in the biodegradable and bioabsorbable polymer and absorbability in the living body. In particular, bioceramic powder having a particle diameter on the order of 0.1 to 5 µm is preferably used for its good bioabsorbability in addition to its unlikeliness to break the coating films.

The bioceramic powder is preferably contained in the biodegradable and bioabsorbable polymer complex 3 by 50 to 90 percent by volume. The polymer complex containing the bioceramic powder at a rate within this range promotes the inductive (conductive) growth of bone tissue by the action of osteoinductivity or osteoconductivity of the bioceramic powder so that the bone tissue is formed rapidly into the superficial portions 1a of the organized structure 1 and the organized structure 1 is joined to the vertebral bodies 20 early. If the content of the bioceramic powder is less than 50 percent by mass, the inductive (conductive) growth of bone tissue proceeds at a slow rate, whilst if the content exceeds 90 percent by mass, the coating films are formed fragile and an excessive amount of bioceramic powder may spill from the superficial portions 1a of the organized structure 1. Thus, neither content is preferred. A further preferred content of the bioceramic powder is in a range of 60 to 80 percent by volume.

The biodegradable and bioabsorbable polymer complex 3 may contain, in addition to the bioceramic powder, an appropriate amount of various cytokines and chemicals that have osteoinductivity, or of an osteoinductive factor such as a BMP (bone morphogenetic protein, a PRP (platelet-rich-plasma), or a BMC (bone mallow cell). In this case, osteoinduction is enhanced such that the growth and replacement of bone tissue in the superficial portions 1a of the organized structure 1 is fostered noticeably, thereby advantageously encouraging early joint of the organized structure 1 with the vertebral bodies 20. In addition, it is also preferred that both the superficial portions 1a of the organized structure 1 be subjected to an oxidizing treatment such as corona discharge, plasma treatment, or hydrogen peroxide treatment to improve the wettability, so as to encourage the effective invasion and growth into the superficial portions 1a of the osseous cells to be proliferated.

The biodegradable and bioabsorbable polymer complex 3 is preferably coated to a thickness on the order of 0.5 to 3 mm. The thickness on this order is effective in causing the fiber structure 1 to be joined to the vertebral bodies 20 solidly, as well as in preventing a biogenic bone from invading as far as the inner layer portion (core layer portion) of the organized structure 1, thereby enabling the fiber structure 1 to deform biomimetically and fulfill satisfactorily its function as an artificial intervertebral disc. If the thickness of the coating layers of the complex 3 is less than 0.5 mm, the bone tissue will not grow inductively (conductively) to an enough thickness, and the intertwinement of the organic fibers with the bone tissue will be insufficient, hence leading to lowered joining strength between the organized structure I and the vertebral bodies 20. Meanwhile, if the thickness of the coating layers of the complex 3 is more than 3 mm, the inner layer portion of the organized structure 1, in which portion bone tissue shall not be formed inductively (conductively), i.e., the inner layer portion that should be capable of deforming biomimetically, will have a relatively reduced thickness and a biogenetic bone may therefore invade as far as the core layer portion of the organized structure, with the result that the fiber structure 1 becomes unable to deform sufficiently and the function as an artificial intervertebral disc deteriorates. A further preferred thickness of the coating layers of the complex 3 is in a range of 1 to 2.5 mm.

The thickness of the coating layers of the biodegradable and bioabsorbable polymer complex 3 may be easily controlled by adjusting the viscosity of the aforementioned suspension that is coated and so forth on the two surfaces of the organized structure 1 or by regulating, e.g., the amount of coating.

The superficial portions 1a on the upper and lower surfaces of the organized structure 1, which portions comprise the coating layers of the biodegradable and bioabsorbable polymer complex 3, have a thickness on the order of 1 to 4 mm and is formed into a soft layer that is softer than the other portion (inner layer portion) of the organized structure 1. Accordingly, the biomaterial 10 for an artificial cartilage is put into an interbody with the vertebral bodies drawn slightly apart and the vertebral bodies are then returned to their original heights to install the biomaterial 10 in the interbody, whereupon the soft superficial portions 1a (soft layers) of the organized structure 1 follow the terrains of the contact surfaces of the superior and inferior vertebral bodies (the inferior surface of the superior vertebral body and the superior surface of the inferior vertebral body) and deform their shapes with ease, so as to be in adhesion with the superior and inferior vertebral bodies.

The superficial portions 1a on the upper and lower sides of the organized structure 1 may have approximately the same softness and thicknesses. As shown in Figs. 3 and 4A to 4C, however, in the biomaterial 10 for an artificial cartilage, the superficial portion 1a on the upper surface of the organized structure 1 to contact the deeply recessed inferior surface of the superior vertebral body 20 has greater softness and thickness in comparison with the superficial portion 1a on the lower surface to contact the substantially flat superior surface of the inferior vertebral body 20. Thus, the superficial portion 1a on the upper surface that is greater in softness and thickness is capable of easily following the deeply recessed inferior surface of the superior vertebral body 20 to compressingly deform greatly into tight contact with the inferior surface, whereas the superficial portion 1a on the lower surface that is less in softness and thickness is capable of following the substantially flat superior surface of the inferior vertebral body 20 to deform a little into tight contact with the superior surface. Specifically, a suitable thickness of the superficial portion 1a on the upper surface is on the order of 2 to 4 mm, and a suitable thickness of the superficial portion 1a on the lower surface is on the order of 1 to 2 mm.

For making the superficial portions 1a soft, as described above, it is effective to, e.g., reduce the thickness or the number of the threads along the Z axis of the threads (organic fibers) along the X, Y, and Z axes in the three-axial and three-dimensional structure, and the softness of the superficial portions 1a on the upper and lower surfaces may be changed freely by selecting by how much thickness or by how many number the threads be reduced.

The superficial portions 1a on the upper and lower surfaces of the organized structure 1 may have flat surfaces, but it is preferred that the superficial portion 1a on the upper surface of the organized structure 1 to contact the deeply recessed inferior surface of the superior vertebral body 20 be raised so as to approximately match the deeply recessed inferior surface of the superior vertebral body 20. The superficial portion 1a on the upper surface of the organized structure 1 thus raised meets the deeply recessed inferior surface of the superior vertebral body 20 in a condition that the superficial portion 1a fits snugly in the inferior surface. This provides an advantage that the pressure is applied from the superior vertebral body 20 approximately uniformly to the organized structure 1, and also an advantage that the raised superficial portion 1a on the upper surface prevents the organized structure 1 from being misaligned or dissociated. Meanwhile, the superficial portion 1a on the lower surface of the organized structure 1 to contact the superior surface of the inferior vertebral body 20 may be approximately flat, for the superior surface of the vertebral body 20 is normally almost flat as compared with the inferior surface.

The pins 2 provided inside the organized structure 1 are made from a lactic acid-based biodegradable and bioabsorbable polymer such as crystalline poly(L-lactic acid), and particularly preferably used is one imparted with increased strength by aligning orientations of polymer molecules and crystals through forging or drawing. The pins 2 have a length that is substantially equal to the thickness of the organized structure 1, and both the ends of the pins 2 are formed into a conical shape of about 0.3 to 2 mm in height. Thus, before the biomaterial 10 for an artificial cartilage constituted by the organized structure 1 is inserted between the vertebral bodies 20, as shown in Figs. 1 and 3, the two ends of each pin 2 do not project from the two surfaces of the organized structure 1. When the biomaterial is inserted between the vertebral bodies 20 and the organized structure 1 is compressed from above and below, as shown in Figs. 2, 4B, and 4C, the two ends of each pin 2 become projected from the two surfaces of the organized structure 1 for about 1 to 2 mm to get into the vertebral bodies 20, whereby the misalignment or dissociation of the biomaterial 10 is reliably prevented. The dimension of the projected portions of the two ends of each pin 2 is preferably in a range of 1 to 2 mm as mentioned above, and the thickness of the pins 2 is preferably in a range of 1 to 3 mm in diameter in order to keep the pins from yielding to the pressure from the vertebral bodies. Note that the two ends of each pin 2 may project slightly from the upper and lower surfaces of the organized structure 1 from the beginning.

The number of pins 2 may be one; however, providing the pin by one is disadvantageous in that, although the pin can prevent the misalignment of the biomaterial 10 in a lateral direction, the pin cannot prevent the rotation of the biomaterial 10. For this reason, it is preferred that, e.g., the pins be provided by two in a biomaterial for a cervical vertebra and by three in a biomaterial for a lumbar vertebra. In particular, providing three pins 2 is advantageous in that the biomaterial 10 can be installed stably between the superior and inferior vertebral bodies 20 with three-point support.

Note that the pins 2 may contain an appropriate amount of the aforementioned bioceramic powder, various cytokines, and the like.

In the case where the biomaterial 10 for an artificial cartilage thus constructed is used as a whole replacement type artificial intervertebral disc to be inserted, as shown in Figs. 2 and 4A to 4C, between the superior and inferior vertebral bodies 20 of a vertebral column (lumbar vertebrae) from the venter side, the soft superficial portions 1a of the organized structure 1 are compressed by the superior and inferior vertebral bodies 20 bearing thereon, whereupon the two ends of each pin 2 become projected from the two surfaces of the organized structure 1 to stick into the superior and inferior vertebral bodies 20, so that the biomaterial is fixed between the vertebral bodies 20 in a free-standing manner without causing misalignment or dissociation. The biomaterial 10 for an artificial cartilage thus fixed in a free-standing manner satisfactorily fulfills its function as an artificial intervertebral disc with the soft superficial portion 1a (soft layer) on the upper surface of the organized structure 1 deforming its shape greatly by following the deeply recessed inferior surface of the superior vertebral body 20 to fit therein while the soft superficial portions 1a on the upper and lower sides deforming a little along the fine recesses and projections on the contact surfaces (inferior and superior surfaces) of the superior and inferior vertebral bodies 20 into adhesion with the superior and inferior vertebral bodies 20, resulting in a condition as shown in Fig. 4C where the organized structure 1 deforms its shape biomimetically, following the movements including anteroposterior bending and lateral bending of the vertebral bodies 20.

Meanwhile, in the biodegradable and bioabsorbable polymer complex 3 coated on the organic fibers of the superficial portions 1a on the two surfaces of the organized structure 1, the bioceramic powder that is exposed on the surfaces of the polymer complex directly promotes osteoconduction. Further, the polymer complex contacts humor that has permeated through the superficial portions 1a, whereby the hydrolysis of the biodegradable and bioabsorbable polymer progresses, and the bioceramic powder that has exposed gradually over the progress of the hydrolysis exerts osteoinductivity or osteoconductivity to cause bone tissue to grow inductively (conductively) into the superficial portions 1a of the organized structure 1 from the vertebral bodies 20. At a final stage, the biodegradable and bioabsorbable polymer is replaced with the bone tissue, and the superficial portions 1a of the organized structure I are solidly joined and fixed to the vertebral bodies 20. After the organized structure 1 is solidly joined to the vertebral bodies 20, the pins 2 also degrade and are absorbed until disappearance.

The biomaterial 10 for an artificial cartilage is not laminated on its two surfaces with plates made of a bioceramic powder-containing biodegradable and bioabsorbable polymer complex as can be seen in the biomaterial of Patent Document 1. Instead, in the biomaterial 10, the organic fibers of the superficial portions 1a of the organized structure 1 are coated with the bioceramic powder-containing biodegradable and bioabsorbable polymer complex 3 as described above, and the soft superficial portions 1a of the organized structure 1 mate tightly with the superior and inferior vertebral bodies 20 (in other words, dead space is not formed between the present biomaterial 10 and a vertebral body, especially between the biomaterial 10 and the recessed portion of the inferior surface of the superior vertebral body 20); therefore, fragments are not produced by the pressure or movement of the vertebral bodies 20, hence reliably eliminating the risk of development of possible harmful effects such as affecting on nerve roots due to scattering of fragments. After the superficial portions 1a on the two surfaces of the organized structure 1 adhere to the superior and inferior vertebral bodies 20 as described above, bone tissue is rapidly formed inductively (conductively) into the superficial portions 1a of the organized structure 1, and the bone tissue thus ingrown inductively (conductively) intertwines with the organic fibers of the superficial portions 1a. Consequently, the organized structure 1 is joined to the vertebral bodies 20 early with great joining strength.

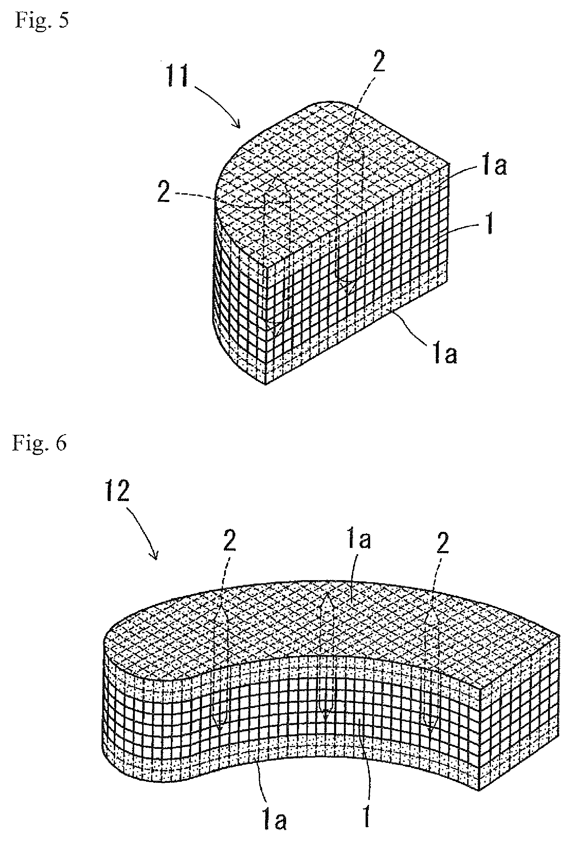

Next, a biomaterial for an artificial cartilage according to another embodiment of the present invention is described with reference to Fig. 5.

The biomaterial 11 for an artificial cartilage is used as a partial replacement type artificial intervertebral disc to be replaced with a half of an intervertebral disc of a vertebral column (a lumbar vertebra) and has such a shape that the above-described biomaterial 10 for an artificial cartilage of a whole replacement type is split into two, right and left pieces. The structure of the biomaterial 11 for an artificial cartilage is substantially the same as that of the above-described biomaterial 10 for an artificial cartilage, namely, the organic fibers of the soft superficial portions 1a on the two surfaces of the organized structure 1 are coated with the biodegradable and bioabsorbable polymer complex containing bioactive bioceramic powder, and the two biodegradable and bioabsorbable pins 2 are provided inside the organized structure 1 in such a manner that, upon compression on the organized structure 1 from superior and inferior vertebral bodies, the two ends of each pin 2 become projected from the two surfaces of the organized structure 1 to stick into the superior and inferior vertebral bodies.

Such a biomaterial 11 for an artificial cartilage of a partial replacement type can be inserted from the dorsum of a vertebral column (lumbar vertebrae) into one side of an interbody, which facilitates surgical operation in comparison with the case of using the biomaterial 10 for an artificial cartilage of a whole replacement type that is inserted into an interbody from the venter side of a vertebral column (lumbar vertebrae). The biomaterial 11 for an artificial cartilage inserted into an interbody is, like the above-described biomaterial 10 for an artificial cartilage, fixed in a free-standing manner by means of the pins 2 without causing misalignment or dissociation, the organized structure 1 has a biomimetic deforming property that is similar to that of a natural intervertebral disc, the soft superficial portions 1a on the two surfaces of the organized structure 1, in which portions the organic fibers are coated with the bioceramic powder-containing biodegradable and bioabsorbable polymer complex, mate tightly with the superior and inferior vertebral bodies, bone tissue is formed inductively (conductively) into the superficial portions 1a early so as to provide direct, solid joint to the vertebral bodies, and no adverse effect is developed due to generation and scattering of fragments. The biomaterial 11 has a plenty of advantages including the aforementioned ones as obtained by the above-described biomaterial 10, and is thus highly suitable as an artificial intervertebral disc of a partial replacement type.

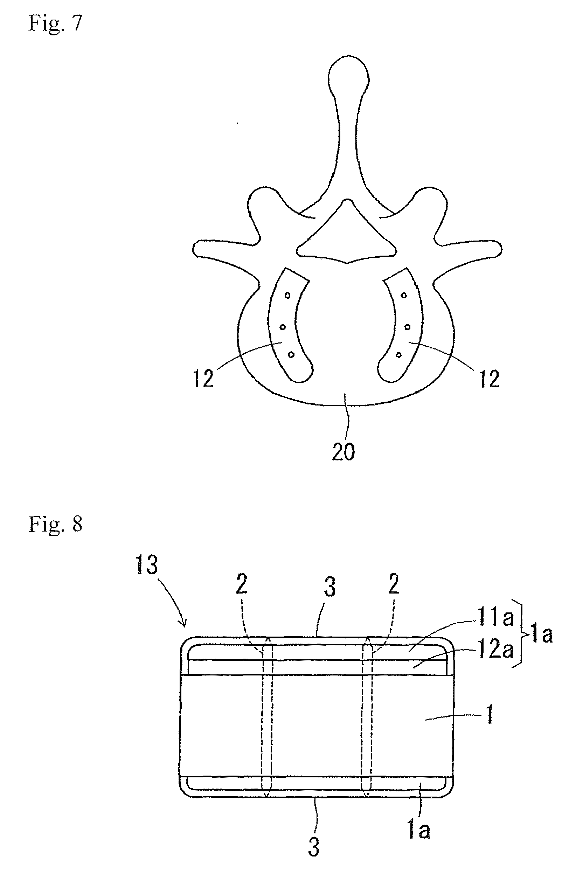

A biomaterial for an artificial cartilage according to still another embodiment of the present invention is described next with reference to Figs. 6 and 7.

The biomaterial 12 for an artificial cartilage of a partial replacement type has a circular arc shape as shown in Fig. 6 with its one end (tip) formed circularly. As shown in Fig. 7, the biomaterials 12 are used in pairs to be installed on the right and left sides in an interbody of a vertebral column (especially, lumbar vertebrae). A standard size of the biomaterial 12 for an artificial cartilage is, in the case of use as, e.g., an artificial intervertebral disc for an adult lumbar vertebra, such that the lateral width dimension is on the order of 9 mm, the thickness dimension is on the order of 11 mm, the radius of curvature with respect to the centerline of the circular arc portion is on the order of 22 to 23 mm, and the length dimension along the centerline of the circular arc portion is on the order of 30 mm.

Although different in shape from the above-described biomaterial 10 for an artificial cartilage of a whole replacement type, the biomaterial 12 for an artificial cartilage is substantially the same in structure as the biomaterial 10. Specifically, the organic fibers of the soft superficial portions 1a on the two surfaces of the organized structure 1 are coated with the biodegradable and bioabsorbable polymer complex containing the bioactive bioceramic powder, and the three biodegradable and bioabsorbable pins 2 are provided inside the organized structure 1, and, upon compression on the organized structure 1 from superior and inferior vertebral bodies, the two ends of each pin 2 become projected from the two surfaces of the organized structure 1 to stick into the superior and inferior vertebral bodies.

Since the biomaterials 12 for an artificial cartilage of a partial replacement type are, as shown in Fig. 7, installed in pairs on the right and left sides of an interbody between vertebral bodies 20 from the dorsum of a vertebral column (lumbar vertebrae), surgical operation can be carried out more easily as compared with the case of using the biomaterial 10 for an artificial cartilage of a whole replacement type. Moreover, since the tip of the biomaterial 12 for an artificial cartilage is formed circularly, the biomaterial 12 can be inserted smoothly into an interbody without being caught at the tip by a vertebral body 20. Like the above-described biomaterials 10 and 11 for artificial cartilages, the biomaterial 12 for an artificial cartilage is also fixed in a free-standing manner by means of the pins 2 without causing misalignment or dissociation, the organized structure 1 deforms biomimetically in such a manner as to simulate a natural intervertebral disc, the soft superficial portions 1a on the two surfaces of the organized structure 1 wherein the organic fibers are coated with the bioceramic powder-containing biodegradable and bioabsorbable polymer complex mate tightly with the superior and inferior vertebral bodies, bone tissue is rapidly formed inductively (conductively) into the superficial portions 1a early so as to provide solid direct joint to the vertebral bodies, and no harmful effect is developed due to the generation and scattering of fragments. The biomaterial 12 has a plenty of advantages including the aforementioned ones as obtained by the biomaterials 10 and 11 to function satisfactorily as an artificial intervertebral disc of a partial replacement type.

A biomaterial for an artificial cartilage according to still another embodiment of the present invention is described next with reference to Fig. 8.

The biomaterial 13 for an artificial cartilage is used as an artificial intervertebral disc of a whole replacement type and is structured such that the soft superficial portion 1a on the upper surface of the organized structure 1 is constituted by two soft layers 11a and 12a, the outer soft layer 11a (the upper superficial portion) of the two layers being even more softer than the inner soft layer 12a (the lower superficial portion). The inner soft layer 12a is approximately as soft as the soft superficial portion 1a on the lower surface of the organized structure 1. The softness of the soft layers 11a and 12a and the superficial portion 1a on the lower surface of the organized structure 1 is controlled, e.g., by choosing by how much thickness or by how many number the threads along the Z axis of the threads (organic fibers) along the X, Y, and Z axes in the three-axial and three-dimensional structure be reduced.

Although the thicknesses of the soft layers 11a and 12a are not limited, a suitable thickness for each layer is on the order of 1 to 2 mm (a total of about 2 to 4 mm). With the thicknesses on this order, the biomaterial 13 for an artificial cartilage is capable of mating tightly with superior and inferior vertebral bodies when inserted and installed therebetween, with the soft layers 11a and 12a greatly deforming their shapes to fit into the recessed portion of the inferior surface of the superior vertebral body. Meanwhile, a suitable thickness for the superficial portion 1a on the lower surface of the organized structure 1 is on the order of 1 to 2 mm as in the case of the biomaterial 10 for an artificial cartilage. With the thickness on this order, the superficial portion is capable of deforming its shape while following the terrain of the approximately flat superior surface of the inferior vertebral body to mate tightly therewith. A suitable thickness for the inner layer portion (the portion other than the soft layers 11a and 12a and the superficial portion 1a on the lower surface) of the organized structure 1 is on the order of 2 to 8 mm. With the thickness on this order, the biomaterial is capable of deforming biomimetically while maintaining the spacing between the superior and inferior vertebral bodies, providing a satisfactory function as an artificial intervertebral disc.

The other structure of the biomaterial 13 for an artificial cartilage is the same as the biomaterial 10 for an artificial cartilage, and so like members are given like reference numerals in Fig. 8 and overlapping description is not given redundantly.

In addition to the advantages as obtained by the biomaterial 10 for an artificial cartilage, the biomaterial 13 for an artificial cartilage provides an effect that, upon being inserted and installed between superior and inferior vertebral bodies, the two soft layers 11a and 12a constituting the superficial portion 1a on the upper surface follow the deeply recessed inferior surface of the superior vertebral body and greatly deform accordingly, while the softest layer 11a on the outer side follows the fine concave/convex terrain on the deeply recessed inferior surface of the vertebral body and deforms a little accordingly, so that the adhesion of the biomaterial to the inferior surface of the vertebral body is further improved and the inductive (conductive) growth of bone tissue into the superficial portion 1a on the upper surface of the organized structure 1 proceeds further rapidly, thereby providing earlier joint to the vertebral bodies.

In the biomaterial 13 for an artificial cartilage, the superficial portion 1a on the upper surface of the organized structure 1 is constituted from the two soft layers 11a and 12a. Obviously, it is also possible to constitute the superficial portion 1a with three or more soft layers, with the softness of the layers set such that the uppermost soft layer is the softest and an inner soft layer is lower in softness than its outer layer. Further, the superficial portion 1a on the lower surface of the organized structure 1 may likewise be constituted with a plurality of soft layers, with the lowermost soft layer formed as the softest layer.

While the above-described biomaterials 10, 11, 12, and 13 for artificial cartilages each have a soft superficial portion 1a (soft layer) on the lower surface of the organized structure 1, the soft superficial portion 1a on the lower surface does not have to be provided. Even if this superficial portion is not provided, the superior surface of the vertebral body to be contacted by the lower surface of the organized structure 1 is substantially flat, and the organized structure 1 is capable of deforming its shape more or less with its inherent softness to match approximately the substantially flat superior surface of the vertebral body. Hence, the inductive (conductive) growth of bone tissue will not be inhibited.

### INDUSTRIAL APPLICABILITY

The biomaterials for artificial cartilages according to the present invention are fixed in a free-standing manner in interbodies by means of pins, mate tightly with the contact surfaces of vertebral bodies and are joined directly to the vertebral bodies early, maintain biomimetic dynamic deforming behavior over a long period of time, and develop no harmful effect due to scattering of fragments of biodegradable and bioabsorbable polymer; the biomaterials are thus highly useful as substitute materials for, e.g., intervertebral discs of living bodies.

## Claims

1. A biomaterial for an artificial cartilage, comprising:
an organized structure comprising organic fibers arranged in one of a multi axial three-dimensional woven or knitted structure having three or more axes and a combined structure of the woven structure and the knitted structure; and
a biodegradable and bioabsorbable pin provided inside the organized structure,
two ends of the pin becoming projected from upper and lower surfaces of the organized structure upon compression on the organized structure from above and below,
each of the upper and lower surfaces or either one of the surfaces of the organized structure having a superficial portion of a soft layer that is softer than the other portion of the organized structure, part or all of the organic fibers of the superficial portion being coated with a biodegradable and bioabsorbable polymer complex containing bioactive bioceramic powder.

2. The biomaterial for an artificial cartilage according to claim 1, wherein the layer coated with the biodegradable and bioabsorbable polymer complex has a thickness in a range of 0.2 to 3 mm.

3. The biomaterial for an artificial cartilage according to claim 1 or 2, wherein the superficial portion on the upper surface of the organized structure is approximately as soft as or softer than the superficial portion on the lower surface.

4. The biomaterial for an artificial cartilage according to claim 1 or 2, wherein at least the superficial portion on the upper surface of the organized structure comprises a plurality of soft layers, and the uppermost soft layer thereof is the softest.

5. The biomaterial for an artificial cartilage according to claim 1 or 2, wherein each of the superficial portions on the upper and lower surfaces or the superficial portion on either one of the surfaces of the organized structure is raised or recessed in such a manner as to follow the terrain of a contact surface of a living body.

6. The biomaterial for an artificial cartilage according to claim 1 or 2, wherein the biodegradable and bioabsorbable polymer complex contains the bioceramic powder by 50 to 90 percent by volume.

7. The biomaterial for an artificial cartilage according to claim 1 or 2, wherein the biodegradable and bioabsorbable polymer of the biodegradable and bioabsorbable polymer complex is any of poly(D,L-lactic acid), a copolymer of L-lactic acid and D,L-lactic acid, a copolymer of lactic acid and glycolic acid, a copolymer of lactic acid and p-dioxanone, a copolymer of lactic acid and ethylene glycol, and a copolymer of lactic acid and caprolactone that is used alone or as a mixture of two or more thereof.

8. The biomaterial for an artificial cartilage according to claim 1, the biomaterial being inserted and installed as an artificial intervertebral disc into an interbody to be joined directly to superior and inferior vertebral bodies adjacent to the biomaterial.
